# EUROPEAN PATENT APPLICATION

(11) **EP 2 689 752 A1**
(43) Date of publication of application: **29.01.2014**
(21) Application number: 12759889.4
(22) Date of filing: 07.03.2012
(51) Int. Cl.: A61F 2/82, A61L 31/00

(54) **STENT**

(30) Priority: 22.03.2011 JP 2011062518
(71) Applicant: Terumo Kabushiki Kaisha, Tokyo 151-0072 (JP)
(72) Inventor: TADA, Yuichi, Ashigarakami-gun Kanagawa 259-0151 (JP)
(74) Representative: Dossmann, Gérard
(86) International application number: PCT/JP2012/055771
(87) International publication number: WO 2012/128032

(57) **Abstract**

Provided is a stent capable of retarding or preventing occlusion that results from granulation. The stent (10) includes: an outer annular body (11) with a circular cross section in which an outer through-hole (14) penetrating in a direction that intersects with the radial direction of the circular cross section is formed and which can expand and contract in the radial direction; and an inner annular body (12) which is disposed on the inside of the outer through-hole (14), forms a double annular cross section together with the outer annular body (11) at least at the openings of the outer through-hole (14), and in which an inner through-hole (15) penetrating in a direction that intersects with the radial direction of said circular cross-section is formed. When placed indwelling in a living body, the outer annular body (11) can expand in the radial direction and can thereby support the living tissue with at least the opening of the inner through-hole (15) of the inner annular body (12) spaced from the living tissue.

## Description

### Technical Field

The present invention relates to a stent to be placed indwelling in a stenosed part or occluded part generated in a living body lumen or indwelling in a hole formed in a living body by an operation so as to maintain a patent state of the part or the hole. Particularly, the invention relates to a stent capable of retarding or preventing occlusion that arises from formation of granulation after placement of the stent.

### Background Art

In general, a stent is a tubular or ring-shaped medical instrument which is capable of radially expanding and contracting, is put indwelling in a stenosed or occluded part or in a hole formed by an operation, and expands to thereby keep the patent state of the part or the hole. For instance, a stent described in Patent Document 1 has a double structure including a tubular main stent and another stent which is bulged radially outward and is disposed to surround the main stent. The main stent expands to make close contact with the inner wall of a lumen, and the surrounding stent presses the inner wall of the lumen, whereby a patent state of the lumen is maintained, while preventing the stent from being detached from the indwelling position.

### Prior Art Document

### Patent Document

Patent Document 1: Japanese Patent Laid-open No. 2009-178545

### Summary of Invention

### Technical Problem

However, such a stent has a problem as follows. Since the stent maintains the patent state by making close contact with the living body, the stent stimulates the living tissue at the contact part between the stent and the living body, particularly the opening edge parts at both ends of the stent, thereby leading to easy formation of granulation. Consequently, the granulation may grow to occlude the lumen or the like.

The present invention has been made in order to solve the above-mentioned problem. Accordingly, it is an object of the present invention to provide a stent capable of retarding or preventing occlusion that arises from granulation.

### Technical Solution

In order to attain the above object, according to the present invention, there is provided a stent including: an outer annular body having an annular cross section, being formed with an outer through-hole penetrating in a direction that intersects with the radial direction of the annular cross section, and being capable of expanding and contracting in the radial direction; and an inner annular body disposed inside the outer through-hole, forming a double annular cross section together with the outer annular body at least at an opening of the outer through-hole, and formed with an inner through-hole penetrating in a direction that intersects with the radial direction of the annular cross section, wherein when placed indwelling in a living body, the outer annular body can expand in the radial direction and can thereby support a living tissue, with at least an opening of the inner through-hole of the inner annular body spaced from the living tissue.

### Advantageous Effects

The stent according to the present invention configured as above-mentioned ensures that even if granulation is formed due to contact of the outer annular body with the living tissue, the inner annular body blocks the granulation while maintaining the patent state by the opening of the inner through-hole spaced from the living tissue. As a result, occlusion arising from granulation can be retarded or prevented.

In addition, a configuration may be adopted wherein the inner annular body protrudes from the opening of the outer through-hole. This ensures that granulation is easily prevented for the inner annular body by the opening of the outer annular body where occlusion is liable to arise from granulation. Therefore, occlusion due to granulation can be retarded or prevented more effectively.

Besides, a configuration may be adopted wherein the outer annular body has a communication hole through which the inside and the outside of the outer through-hole communicate with each other in the radial direction, and a swelling layer which is swellable with water is disposed at an outer surface of the inner annular body. This ensures that the swelling layer swells to clog up the communication hole in the outer annular body, so that it is difficult for the granulation to enter via the communication hole into the inside of the outer annular body and into the inside of the inner annular body. Consequently, occlusion due to granulation can be retarded or prevented more effectively.

In addition, the stent may include a surface lubricating layer which is swellable with water and is disposed at an inner surface of the inner annular body. This configuration ensures that the surface lubricating layer swells to thereby exhibit lubricity, so that dischargeability of secretion such as sputum can be enhanced.

Besides, the stent may include a biologically active agent release layer which contains a biologically active agent, a biodegradable polymer, and a plasticizer and which is formed on at least one of the outer annular body and the inner annular body. This ensures that the biodegradable polymer is decomposed in a living body, whereby the biologically active agent is released. As a result, formation of granulations itself is restrained, and, therefore, occlusion due to granulation can be retarded or prevented more effectively.

In addition, a configuration may be adopted wherein the outer annular body and the inner annular body are each formed from a biodegradable polymer. This ensures that the stent is decomposed and absorbed in a living body, so that semipermanent exertion of mechanical stress on the living tissue can be avoided.

Besides, the stent may include a protrusion formed at an outer surface of the outer annular body. This configuration ensures that the protrusion is locked on the living tissue, so that the stent can be prevented from being detached from the indwelling position.

### Brief Description of Drawings

[FIG. 1]

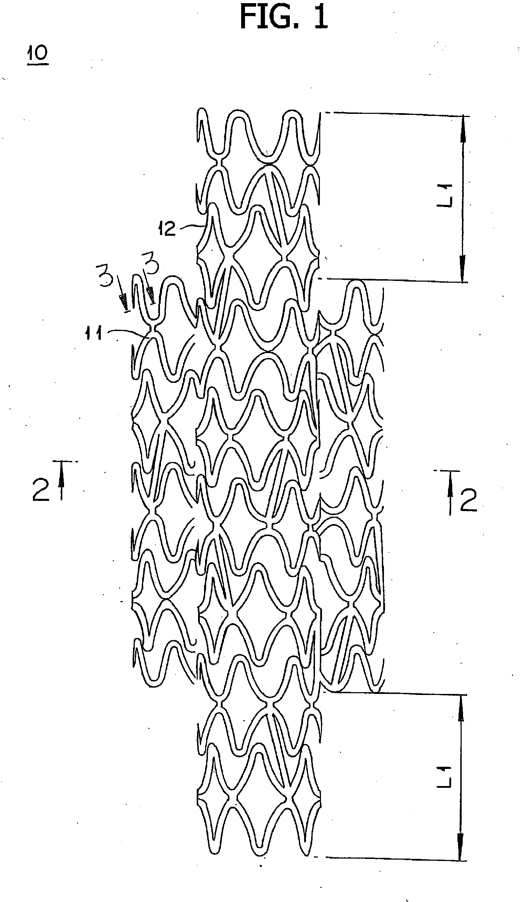

   FIG. 1 is a schematic configuration view of a stent according to a first embodiment.
[FIG. 2]

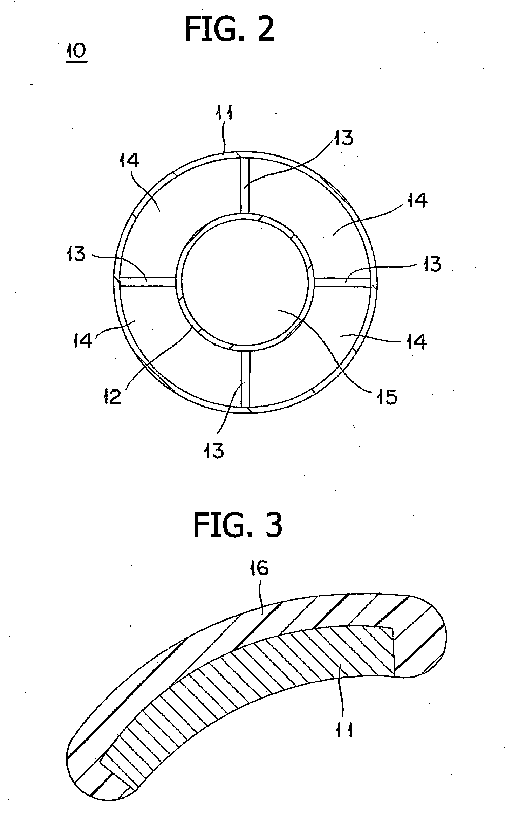

   FIG. 2 is a sectional view taken along line 2-2 of FIG. 1.
[FIG. 3]
   FIG. 3 is a sectional view taken along line 3-3 of FIG. 1.
[FIG. 4]

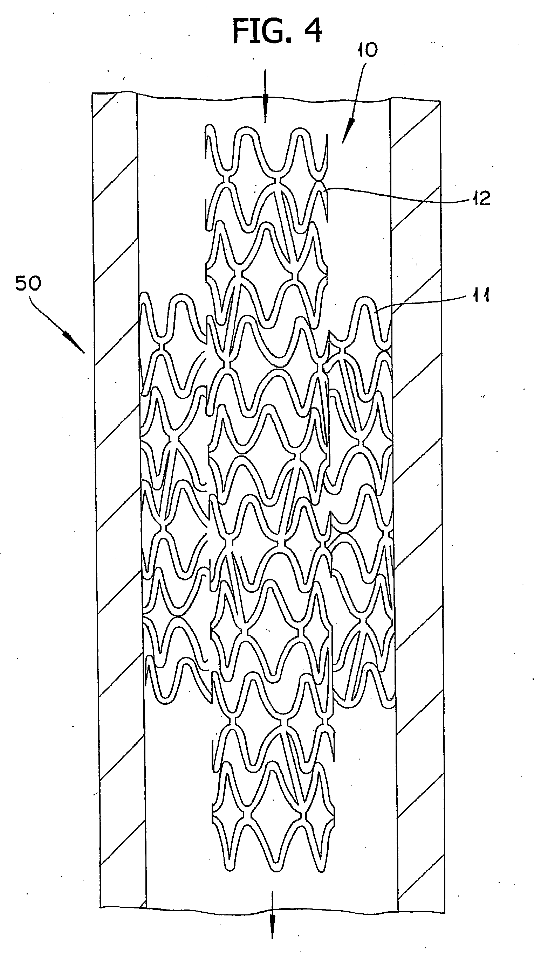

   FIG. 4 illustrates a state in which the stent of the first embodiment is set indwelling in a lumen.
[FIG. 5]

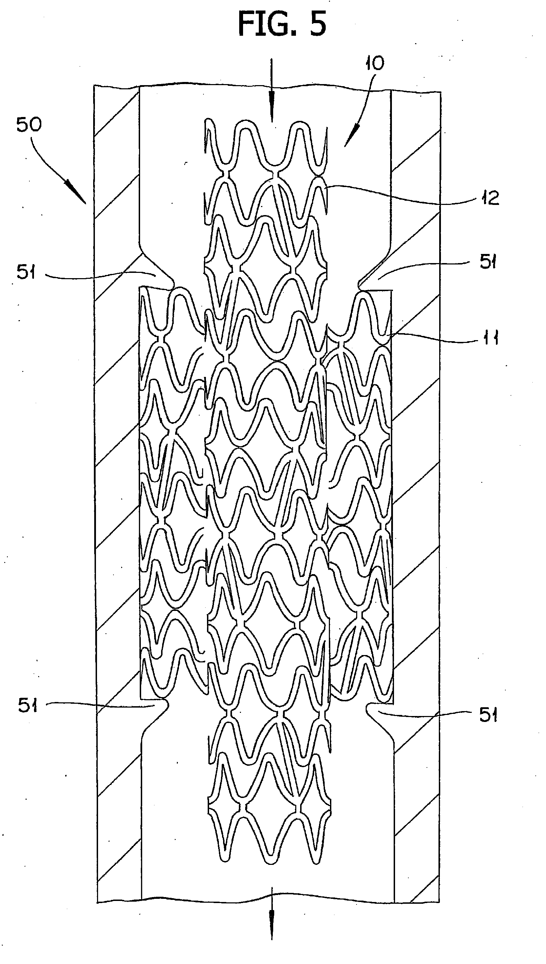

   FIG. 5 illustrates a state in which a granulation is formed in the vicinity of an opening of an outer annular body in the first embodiment.
[FIG. 6]

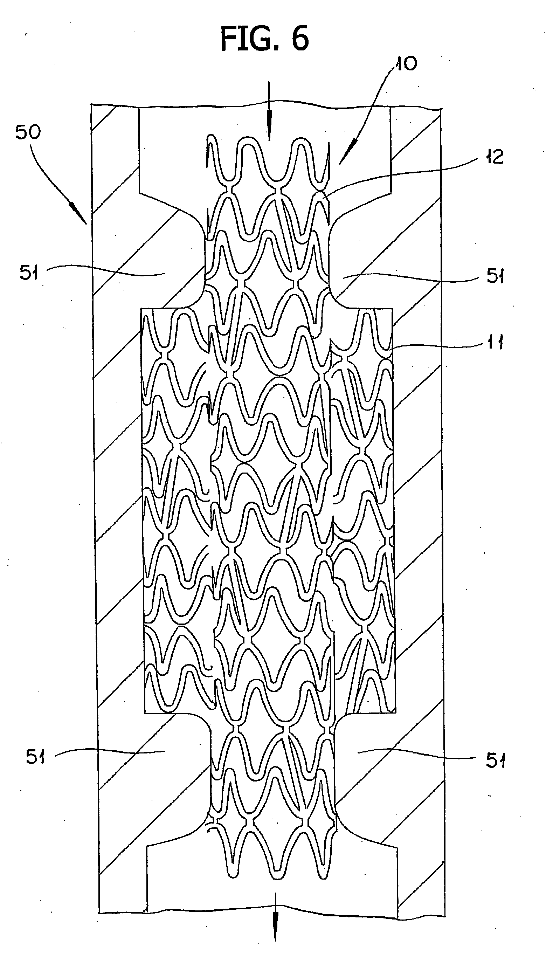

   FIG. 6 illustrates a state in which an inner annular body in the first embodiment has prevented granulation, thereby preventing occlusion.
[FIG. 7]

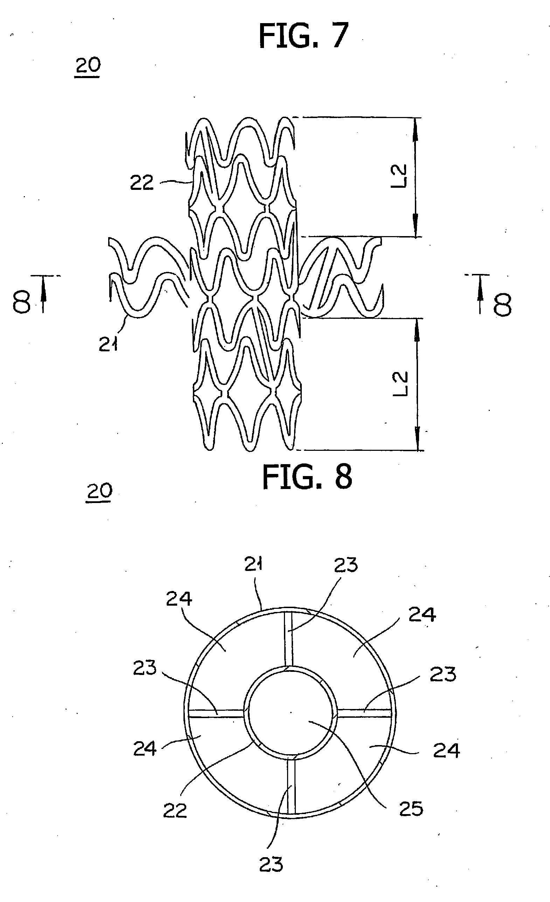

   FIG. 7 is a schematic configuration view of a stent according to a second embodiment.
[FIG. 8]
   FIG. 8 is a sectional view taken along line 8-8 of FIG. 7.
[FIG. 9]

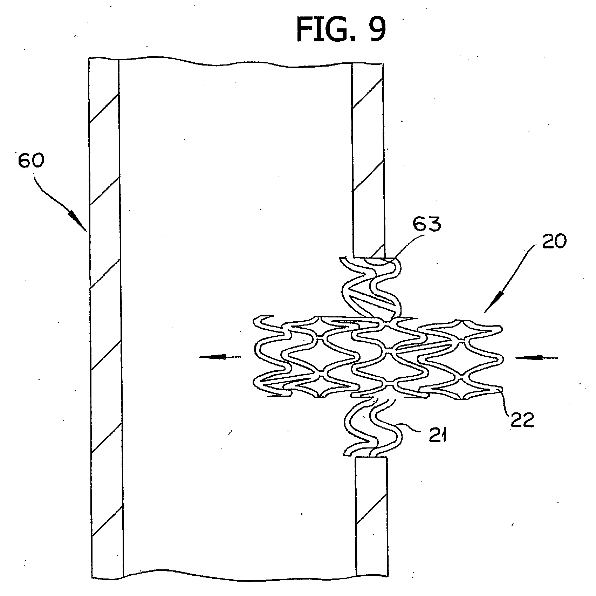

   FIG. 9 illustrates a state in which the stent of the second embodiment is set indwelling in an opening formed in a wall of a lumen.
[FIG. 10]

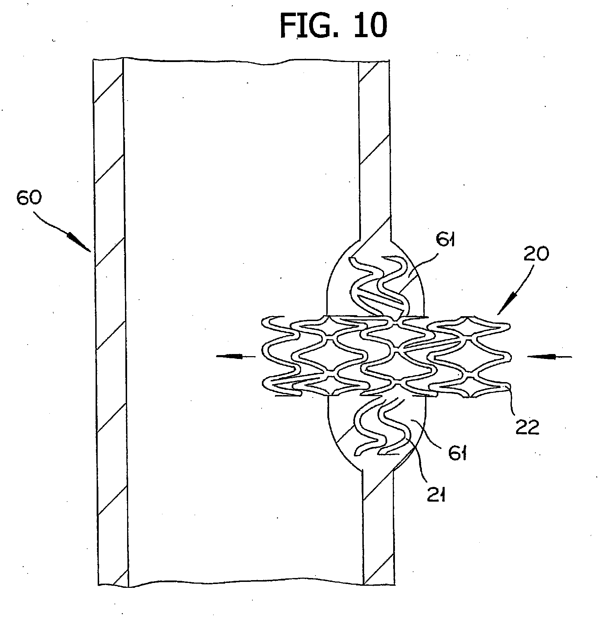

   FIG. 10 illustrates a state in which an outer annular body in the second embodiment is covered with a granulation.
[FIG. 11]

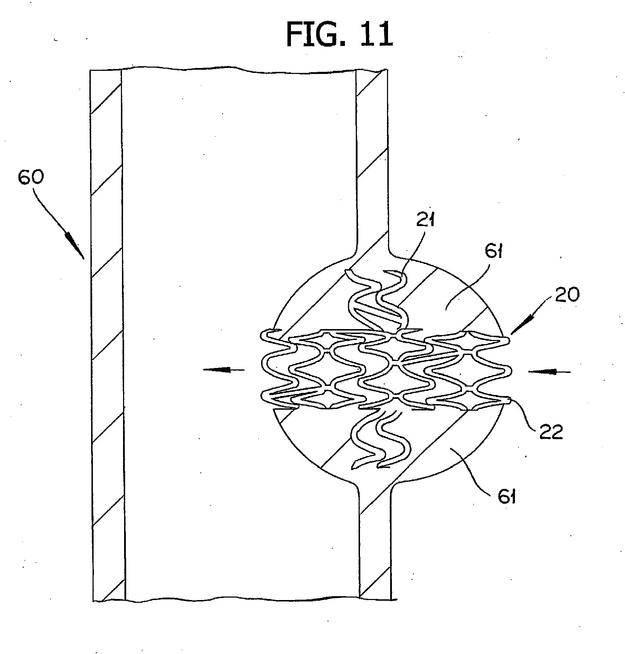

   FIG. 11 illustrates a state in which an inner annular body in the second embodiment has prevented granulation, thereby preventing occlusion.
[FIG. 12]

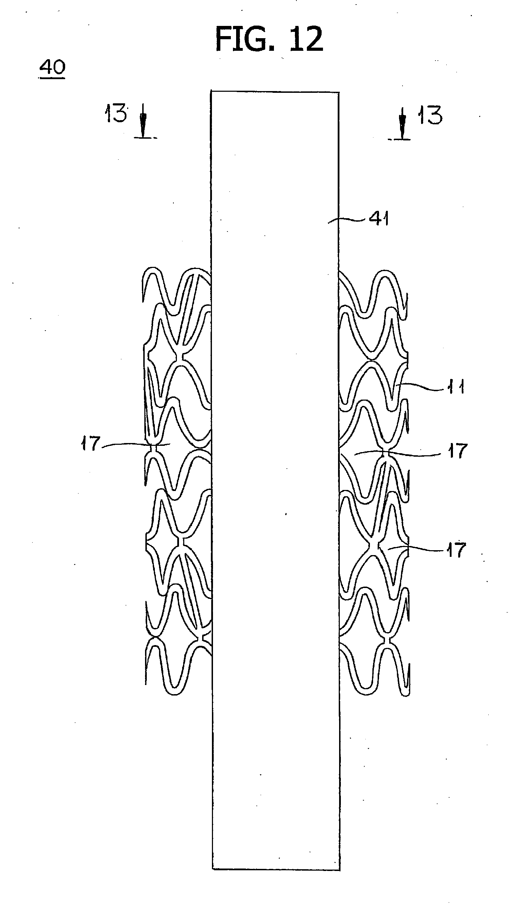

   FIG. 12 is a schematic configuration view of a stent according to a third embodiment.
[FIG. 13]

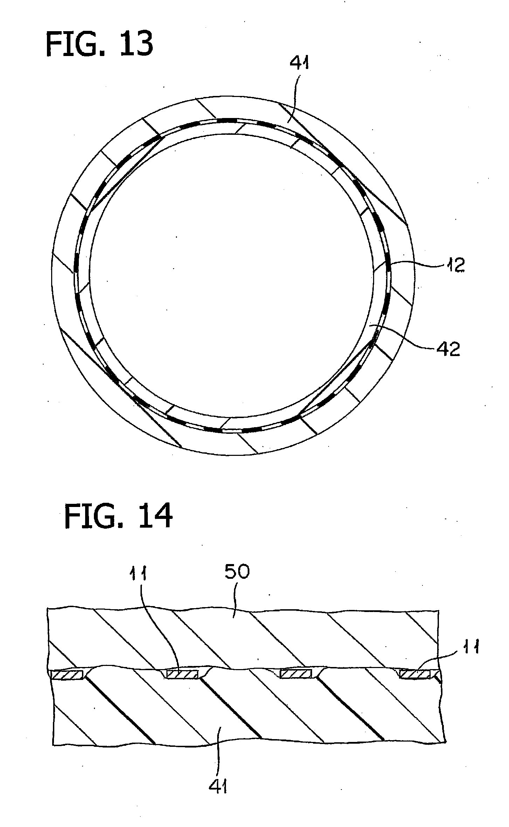

   FIG. 13 is a sectional view taken along line 13-13 of FIG. 12.
[FIG. 14]
   FIG. 14 is a partly enlarged sectional view illustrating a state in which a swelling layer has swelled to clog up openings of an outer annular body.
[FIG. 15]

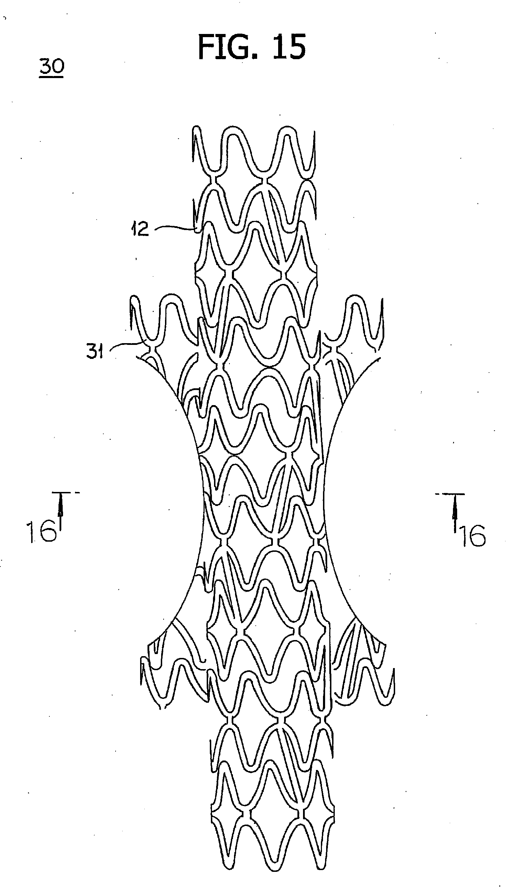

   FIG. 15 is a schematic configuration view of a stent according to a modification.
[FIG. 16]

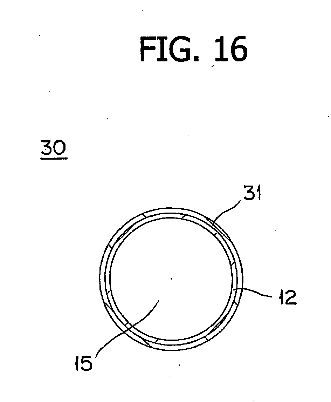

   FIG. 16 is a sectional view taken along line 16-16 of FIG. 15.

### Mode for Carrying Out the Invention

Now, embodiments of the present invention will be described below, referring to the drawings. Incidentally, for convenience of description, the dimensional ratios in the drawings may be exaggerated, so that they may differ from the actual dimensional ratios.

### <First Embodiment>

As shown in FIGS. 1 and 2, a stent 10 according to a first embodiment includes: an outer annular body 11 capable of expansion and contraction in the radial direction; an inner annular body 12 disposed inside the outer annular body 11 and capable of expansion and contraction in the radial direction; and link parts 13 interconnecting the outer annular body 11 and the inner annular body 12. In addition, as shown in FIG. 3, the stent 10 has a biologically active agent release layer 16 formed on a surface of the outer annular body 11.

The stent 10 is used in the state of being set indwelling in a respiratory region inclusive of lumens branched on the peripheral side of a trachea, specifically, in the trachea, main bronchi, lobar bronchi, bronchial tubes, bronchioles, terminal bronchioles, respiratory bronchioles, and alveolar ducts. Particularly, the stent 10 is put indwelling in a stenosed part or occluded part generated at the lumen constituting the respiratory region so as to maintain the patent state of such a part, whereby the ventilatory function of the lung is kept normal.

The outer annular body 11 has a configuration in which a plurality of wavy annular members each formed by putting struts (which are thin wavy linear materials) into an annular shape are aligned in an axial direction and joined to one another. The outer annular body 11 is in a mesh-like form. The outer annular body 11 has a tubular shape, and has an outer through-hole 14 penetrating in the axial direction.

The length of the outer annular body 11 in the axial direction is, for example, 2 to 25 mm. In addition, the outside diameter of the outer annular body 11 when expanded is, for example, 3 to 6 mm, and the inside diameter in the expanded state is, for example, 2.8 to 5.8 mm. The outer annular body 11 is of a self-expandable type such that the outer annular body 11 itself has an expanding-and-contracting function.

The operator preliminarily stores the stent 10 inside a flexible tubular sheath (not shown), with the outer annular body 11 contracted in the radial direction, and then introduces the sheath to a target site in the respiratory region. Thereafter, the operator pushes out the stent 10 from the distal end of the sheath. When the stent 10 is pushed out of the sheath, a force having been exerted on the outer annular body 11 from the sheath is removed, so that the outer annular body 11 expands by its own expanding function, resulting in that the stent 10 is set indwelling in the target site in the respiratory region.

The outer annular body 11 has a strength sufficient for the expanded inner annular body 12 to be kept spaced from the living body, against a pressure exerted from the lumen. The pressure required to contract the outer annular body 11 until the outer annular body 11 makes contact with the expanded inner annular body 12 is measured as a force generated in the radial direction when an arbitrary point in the major axial direction of the stent is contracted. This force is preferably 0.1 to 10 N, more preferably 0.3 to 5.0 N.

The material for forming the outer annular body 11 is preferably a biocompatible material, for example, stainless steels, tantalum or tantalum alloys, platinum or platinum alloys, gold or gold alloys, cobalt-based alloys, cobalt-chromium alloys, titanium alloys, iron-based alloys, niobium alloys, etc. Among the stainless steels, preferred is SUS316L, which is the highest in corrosion resistance. As the titanium alloy, there may be used those metals enhanced in rigidity, such as CNT-added Ni-Ti alloys produced through addition of carbon nanotubes, and oxide-added Ni-Ti alloys produced through addition of an oxide. As the iron-based alloy, there may be used iron-based shape-memory alloys or iron-based superelastic alloys.

In addition, the material for forming the outer annular body 11 is not restricted to the above-mentioned metallic materials, and may be a biodegradable polymer. The biodegradable polymer is, for example, at least one selected from the group including polylactic acid, polylactic acide stereo complex, polyglycolic acid, polylactic acid-polyglycolic acid copolymer, polyhydroxybutyric acid, polymalic acid, poly-α-amino acid, collagen, laminin, heparan sulfate, fibronectin, vitronectin, chondroitin sulfate, hyaluronic acid, and polycaprolactone, or a copolymer of some of them.

Like the outer annular body 11, the inner annular body 12 has a configuration in which a plurality of wavy annular members each formed by putting struts (which are thin wavy linear materials) into an annular shape are aligned in an axial direction and joined to one another. The inner annular body 12 has a mesh-like form and is tubular in shape. Besides, the inner annular body 12 has an inner through-hole 15 penetrating in the axial direction, and forms a double annular cross section together with the outer annular body 11, over the whole length of the outer annular body 11 in the axial direction. In addition, the inner annular body 12 is protruding from openings at both ends of the outer annular body 11.

The length of the inner annular body 12 in the axial direction is, for example, 3 to 30 mm. The outside diameter of the inner annular body 12 when expanded is, for example, 2.4 to 5.4 mm, and the inside diameter in the expanded state is, for example, 2.6 to 5.6 mm. In addition, the axial length L1 of each of those parts of the inner annular body 12 which are protruding from the openings at both ends of the outer annular body 11 is preferably 2 to 10 mm.

The inner annular body 12 is of a balloon-expandable type such that the inner annular body 12 itself does not have an expanding function and it is expanded by an expanding force of a balloon. The operator expands the outer annular body 11 and, thereafter, inflates the balloon inserted in the inner annular body 12, to thereby expand the inner annular body 12.

As the material for forming the inner annular body 12, there may be used, for example, the same or similar materials to those mentioned above as the material for the outer annular body 11. Besides, the material forming the outer annular body 11 and the material forming the inner annular body 12 may be the same or different.

The link parts 13 are linear members, which expand or contract according as the gap between the outer annular body 11 and the inner annular body 12 is varied attendant on expansion/contraction of the annular bodies. The link parts 13 are formed from any of materials which are the same as or similar to the materials for the outer annular body 11 and the inner annular body 12. Where the outer annular body 11, the inner annular body 12 and the link parts 13 are formed from metallic materials, the link part 13 is joined at its ends to the outer annular body 11 and the inner annular body 12 by welding, for example. Alternatively, the link part 13 may be connected to the outer annular body 11 and the inner annular body 12 by hooking its end portions on mesh openings of these annular bodies. Furthermore, the material for the link parts 13 is not restricted to the materials which are the same as or similar to the materials for the outer annular body 11 and the inner annular body 12. For instance, the link parts 13 may be formed from a flexible material, such as fiber.

The biologically active agent release layer 16 is for restraining formation of granulation, and contains a biologically active agent, a biodegradable polymer, and a plasticizer. The biologically active agent is, for example, at least one selected from the group including carcinostatic agents, immunosuppressants, antibiotics, antirheumatics, antithrombogenic agents, HMG-Co reductase inhibitors, ACE inhibitors, calcium antagonists, antihyperlipidemic drugs, anti-inflammatory agents, integrin inhibitors, antiallergic agents, antioxidants, GPIIbIIIa antagonists, retinoids, flavonoids, carotinoids, lipid improving drugs, DNA synthesis inhibitors, tyrosine kinase inhibitors, antiplatelet agents, vascular smooth muscle proliferation inhibitors, anti-inflammatory drugs, bio-derived materials, interferon, and NO production promoting materials. Examples of the biodegradable polymer contained in the biologically active agent release layer 16 include the same materials as those mentioned above in relation to the outer annular body 11. The plasticizer is not specifically restricted, and those plasticizers ordinarily used in the medical field can be used. The plasticizer is, for example, polyglycerine ester. With the plasticizer contained in the biologically active agent release layer 16, flexibility is increased, and the layer can be prevented from peeling or the like.

Now, the operation and effect of the stent 10 will be described below.

As shown in FIG. 4, when the operator sets the stent 10 indwelling in a target site in a lumen 50, the outer annular body 11 supports the lumen 50 from inside, in the state in which the outer annular body 11 is expanded in the radial direction and the whole body of the inner annular body 12 is spaced from the lumen 50. Then, as shown in FIGS. 5 and 6, even if granulations 51 are formed due to stimulation of the living body tissue by the outer annular body 11, the inner annular body 12 blocks the granulations 51 while maintaining a patent state by its inner through-hole 15. Therefore, the stent 10 can retard or prevent occlusion which arises from the granulations 51.

In addition, with the inner annular body 12 protruding from the openings of the outer annular body 11, the inner annular body 12 will easily blocks the granulations 51 at the openings of the outer annular body 11 where the granulations 51 are liable to grow. Accordingly, the stent 10 can more effectively retard or prevent occlusion which is caused by the granulations 51.

Besides, since the stent 10 is used in the respiratory region, clogging-up with thrombus would not occur, unlike in the case where the stent 10 is used in a blood vessel.

In addition, the biologically active agent release layer 16 is decomposed inside the lumen 50, whereby the biologically active agent is released. As a result, formation of the granulations 51 itself is restrained, so that occlusion due to the granulations 51 can be effectively retarded or prevented.

Besides, since the outer annular body 11 and the inner annular body 12 are formed from a biodegradable polymer and the stent 10 is therefore decomposed and absorbed in the lumen 50, semipermanent exertion of a mechanical stress on the lumen 50 can be avoided. In addition, since the formation of the granulations 51 itself is restrained due to the removal of the stimulus given to the living body tissue from the stent 10, occlusion of the lumen 50 can be retarded or prevented.

### <Second Embodiment>

As shown in FIGS. 7 and 8, a stent 20 according to a second embodiment includes: an outer annular body 21 capable of expansion and contraction in the radial direction; an inner annular body 22 disposed inside the outer annular body 21 and capable of expansion and contraction in the radial direction; and link parts 23 interconnecting the outer annular body 21 and the inner annular body 22. In addition, the stent 20 has a biologically active agent release layer (not shown) formed on a surface of the outer annular body 21; however, this layer is the same as in the first embodiment, and, therefore, overlapping description thereof will be omitted.

The stent 20 is used in the state of being left indwelling on a wall of a lumen constituting a respiratory region, in treatment of pulmonary emphysema by the so-called airway bypass. In the emphysema, alveoli located at peripheral ends of the lumen are destructed and expanded largely, so that a stenosed part or occluded part in which a part of the lumen is crushed by the enlarged alveoli is generated. As a result, it becomes difficult for air to go out of the alveoli at the time of expiration. In view of this, the stent 20 is put indwelling in an opening formed in the lumen wall, the opening serving as a bypass between the lumen and the destructed alveoli adjacent to the lumen. Then, the stent 20 maintains the patent state of the opening, thereby playing the role of directly extracting the expiratory air from the alveoli.

The outer annular body 21 has a configuration in which a plurality of wavy annular members each formed by putting struts (which are thin wavy linear materials) into an annular shape are aligned and are joined to one another, like in the first embodiment. The outer annular body 21 has a mesh-like form. In addition, the outer annular body 21 has outer through-holes 24 penetrating in the direction orthogonal to the radial direction. The length of the outer annular body 21 in the direction orthogonal to the radial direction is smaller than that in the first embodiment. In other words, the outer annular body 21 is rather ring-like in shape than tubular in shape like in the first embodiment.

The length of the outer annular body 21 in the direction orthogonal to the radial direction is, for example, 1 to 3 mm. The outside diameter of the outer annular body 21 when expanded is, for example, 3 to 5 mm, and the inside diameter in the expanded state is, for example, 2.8 to 4.8 mm.

The material for forming the outer annular body 21 is the same as in the first embodiment. In addition, the outer annular body 21 is of a self-expandable type such that the outer annular body 21 itself has an expanding and contracting function, like in the first embodiment. Before putting the stent 20 indwelling, the operator preliminarily forms in a lumen wall an opening communicating with the broken alveoli, by use of a wire provided with a needle at the distal end thereof or the like. Then, like in the first embodiment, the operator pushes out the stent 20 from a sheath inserted in the vicinity of the opening, to thereby cause the outer annular body 21 to expand, whereby the stent 20 is put indwelling in the opening formed in the lumen.

The outer annular body 21 has a strength sufficient for the expanded inner annular body 22 to be kept spaced from the living body, against the pressure exerted from the lumen. The pressure required to contract the expanded outer annular body 21 until the outer annular body 21 makes contact with the expanded inner annular body 22 is, for example, preferably 0.1 to 10 N, and more preferably 0.3 to 6.0 N.

The inner annular body 22 has a configuration in which a plurality of wavy annular members each formed by putting struts (which are thin wavy linear materials) into an annular shape are aligned in the axial direction and joined to one another. The inner annular body 22 has a mesh-like form and is tubular in shape. In addition, the inner annular body 22 has an inner through-hole 25 penetrating in the axial direction, and forms a double annular cross section together with the outer annular body 21 over the whole length of the outer annular body 21 in the direction orthogonal to the radial direction. In addition, the inner annular body 22 is protruding from openings of the outer annular body 21.

The length of the inner annular body 22 in the axial direction is, for example, 2 to 5 mm. In addition, the outside diameter of the inner annular body 22 when expanded is, for example, 2.8 to 4.8 mm, and the inside diameter in the expanded state is, for example, 2.4 to 4.5 mm. Besides, the axial length L2 of each of those portions of the inner annular body 22 which protrude from the openings of the outer annular body 21 is preferably 0.5 to 4.2 mm. The material for forming the inner annular body 22 is the same as in the first embodiment. In addition, the link parts 23 are substantially the same as in the first embodiment.

Now, the operation and effect of the stent 20 will be described below.

As shown in FIG. 9, when the operator puts the stent 20 indwelling in an opening 63 formed in the wall of a lumen 60, the outer annular body 21 supports the opening 63 in the state in which it is expanded in the radial direction and the whole part of the inner annular body 22 is spaced from the opening 63. Then, as shown in FIGS. 10 and 11, even if a granulation 61 is formed due to stimulation of the living body tissue by the outer annular body 21, the inner annular body 22 blocks the granulation 61 while keeping a patent state by the inner through-hole 25. Therefore, the stent 20 can retard or prevent occlusion due to the granulation 61.

In addition, with the inner annular body 22 protruding from the opening of the outer annular body 21, the inner annular body 22 will easily prevent the granulation 61 at the opening of the outer annular body 21 where the granulation 61 is liable to grow. Accordingly, the stent 20 can more effectively retard or prevent occlusion due to the granulation 61.

Besides, since the stent 20 is used in the respiratory region, clogging-up due to thrombus would not occur, unlike in the case where the stent 20 is used in a blood vessel.

In addition, the stent 20 has a biologically active agent release layer (not shown), and the layer is decomposed, whereby a biologically active agent is released. As a result, formation of the granulation 61 itself is restrained, so that occlusion due to the granulation 61 can be effectively retarded or prevented.

Besides, since the stent 20 is decomposed and absorbed because the outer annular body 21 and the inner annular body 22 are formed from a biodegradable polymer, semipermanent exertion of a mechanical stress on the living tissue can be obviated. In addition, since the formation of the granulation 61 itself is restrained by the removal of the stimulus given to the living body tissue from the stent 20, it is effective in retarding or preventing occlusion.

### <Third Embodiment>

In outline, as shown in FIGS. 12 and 13, a stent 40 according to a third embodiment includes a water-swellable swelling layer 41 disposed on an outer surface of an inner annular body 12, and a water-swellable surface lubricating layer 42 disposed on an inner surface of the inner annular body 12, in addition to the components according to the first embodiment. In regard of the other components than the swelling layer 41 and the surface lubricating layer 42, the stent 40 is substantially the same as the stent 10; therefore, overlapping descriptions of the other components will be omitted.

The swelling layer 41 is tubular in shape, and covers entirely the outer circumference of the inner annular body 12. As the swelling layer 41, there may be used, for example, a hydrogel obtained by a method in which a hydrophilic monomer having at least one kind of hydrophilic group in the molecule thereof is polymerized in the presence of a crosslinking agent. Examples of the polymerization method include a chemical polymerization method in which a radical polymerization initiator is used, a photopolymerization method in which photopolymerization initiator is used, and a radiation polymerization method. Examples of the polymerization initiators include persulfates such as sodium persulfate, potassium persulfate, or ammonium persulfate; hydrogen peroxide; azo compounds such as azobis-2-methylpropionamidine hydrochloride or azoisobutyronitrile; and peroxides such as benzoyl peroxide, lauroyl peroxide, cumene hydroperoxide or benzoyl oxide, which may be used either singly or in combination of two or more of them. In this instance, there can be used at least one polymerization promoter, examples of which include: reducing agents such as sodium hydrogen sulfite, sodium sulfite, Mohr's salt, sodium pyrobisulfite, formaldehyde sodium sulfoxylate or ascorbic acid; amine compounds such as ethylenediamine, sodium ethylenediaminetetraacetate, glycine, or N,N,N',N'-tetramethylethylenediamine. Examples of the hydrophilic monomer include: (meth)acrylic monomers such as N,N-dimethylacrylamide (DMAA), 2-hydroxyethyl methacrylate (HEMA), (meth)acrylic acid, polyethylene glycol monomethacrylate, and glycerol methacrylate; and hydrophilic vinyl-containing monomers such as N-vinylpyrrolidone (NVP), N-vinyl-N-methylacetamide, N-vinyl-N-ethylacetamide, N-vinyl-N-ethylformamide, and N-vinylformamide, which may be used either singly or in combination of two or more of them. Specific examples of the crosslinking agent include: divinyl compounds such as N,N'-methylenebis(meth)acrylamide, N,N'-(1,2-dihydroxyethylene)-bis(meth)acrylamide, diethyene glycol di(meth)acrylate, (poly)ethylene glycol di(meth)acrylate, triethylene glycol di(meth)acrylate, propylene glycol di(meth)acrylate, trimethylolpropane di(meth)acrylate, trimethylolpropane tri(meth)acrylate, pentaerythritol di(meth)acrylate, pentaerythritol tri(meth)acrylate, pentaerythritol tetra(meth)acrylate, (poly)propylene glycol di(meth)acrylate, glycerine tri(meth)acrylate, glycerine acrylate methacrylate, ethylene oxide-modified trimethylolpropane tri(meth)acrylate, pentaerythritol tetra(meth)acrylate, and dipentaerythritol hexa(meth)acrylate; triallyl cyanurate, triallyl isocyanurate, triallyl phosphate, triallylamine, poly(meth)allyloxy alkanes, (poly)ethylene glycol diglycidyl ether, glycerol diglycidyl ether, ethylene glycol, polyethylene glycol, propylene glycol, glycerine, pentaerythritol, ethylenediamine, polyethyleneimine, glycidyl (meth)acrylate, triallyl isocyanurate, trimethylolpropane di(meth)allyl ether, tetraallyloxyethane, or glycerol propoxytriacrylate. These crosslinking agents may be used either singly or in combination of two or more of them. Other than the hydrogels obtained by polymerization reactions, natural compounds such as hyaluronic acid and collagen can also be used.

The swelling layer 41 swells outward in the radial direction of the inner annular body 12 by absorbing water. Preferably, the swelling layer 41 reaches the mesh openings 17 (communication holes) of the outer annular body 11 to clog up the mesh openings 17. More preferably, the swelling layer 41 comes to the outside of the outer annular body 11 through the mesh openings 17 while clogging up the mesh openings 17. The radial spacing (distance) between the outer annular body 11 and the inner annular body 12 when expanded is, for example, 0.1 to 1.5 mm. In addition, the thickness of the swelling layer 41 is, for example, 0.05 to 1.60 mm.

The surface lubricating layer 42 formed from the same or similar component to that of the surface swelling layer 41 is tubular in shape, and covers entirely the inner circumference of the inner annular body 12. In this instance, the swelling layer 41 and the surface lubricating layer 42 may be different or the same in composition. As the surface lubricating layer 42 swells by absorbing water, the frictional resistance on the surface of the surface lubricating layer 42 is thereby reduced. In addition, when swelled, the surface lubricating layer 42 maintains a patent state of the inner annular body 12. The thickness of the surface lubricating layer 42 is, for example, 0.05 to 1.20 mm.

The stent 40 is placed indwelling in a lumen 50 by the same method as that for the stent 10 in the first embodiment. After the placement of the stent 40, the swelling layer 41 and the surface lubricating layer 42 absorb water which is present inside the lumen, or absorb water which is supplied by the operator by use of a catheter or the like. Then, the swelling layer 41 swells to clog up the mesh openings 17 of the outer annular body 11, as shown in FIG. 14. In addition, the surface lubricating layer 42 exhibits lubricity.

Since the swelling layer 41 thus swells to clog up the mesh openings 17 of the outer annular body 11, it is difficult for the granulation to enter via the mesh openings 17 into the inside of the outer annular body 11 and into the inside of the inner annular body 12. Therefore, the stent 40 according to the third embodiment has an effect of more effectively retarding or preventing occlusion due to granulation, in addition to the effects obtained in the first embodiment. Furthermore, sine the surface lubricating layer 42 displays lubricity, dischargeability of secretion such as sputum is enhanced.

The present invention is not restricted to the above-described embodiments, and various alterations are possible within the scope of the claims.

For instance, the stent is not limited to those for use in the respiratory region, as in the embodiments above; for example, the stent may be one that is to be put indwelling in other lumen in a living body, such as bile duct or urethra.

In addition, while the outer annular body is of the self-expandable type and the inner annular body is of the balloon-expandable type in the embodiments above, this is not restrictive of the present invention. For instance, the present invention embraces those stents in which the outer annular body is of the balloon-expandable type and the inner annular body is of the self-expandable type. Further, the stent may have a configuration in which both the outer annular body and the inner annular body are of the self-expandable type or of the balloon-expandable type.

Besides, the outer annular body and the inner annular body are not restricted to those which are mesh-like in form; for example, a form obtained by providing an outer circumferential wall of a tubular body such as a metallic pipe with a plurality of holes or a coil-like form may also be adopted. The inner annular body is not limited to a tubular body, and may be ring-like in shape. The inner annular body is not restricted to a stent, and may be a tubular body which does not have the radially expanding and contracting function, which is fixed in diameter (radius), which does not have holes such as mesh openings, and in which radial communication is intercepted.

In addition, the stent may have a projection or projections formed on the outer surface of the outer annular body. In this case, when the stent is put indwelling in a lumen, the projection or projections are locked on the living body, so that the stent can be prevented from being detached from the indwelling position.

Besides, the biologically active agent release layer may be formed not on the outer annular body but on the inner annular body, or may be formed on both of the annular bodies.

In addition, the present invention embraces those stents which include no link part for interconnecting the outer annular body and the inner annular body. For instance, a configuration may be adopted wherein as represented by a stent 30 shown in FIG. 15, an outer annular body 31 is so shaped that its radius (or diameter) decreases along directions from both ends in the axial direction toward the center in the axial direction, and, as shown in FIG. 16, the outer annular body 31 is joined directly to the inner annular body 12 at the radially reduced central portion. In this case, the outer annular body 31 sets the inner annular body 12 spaced from the lumen at its radially enlarged portions at both ends in the axial direction.

Furthermore, the present invention is based on Japanese Patent Application No. 2011-062518, filed on March 22, 2011, the whole content of which is incorporated herein by reference.

### Explanation of Reference Numerals

- 10, 20, 30, 40: Stent,
- 11, 21, 31: Outer annular body,
- 12, 22: Inner annular body,
- 13, 23: Link part,
- 14, 24: Outer through-hole,
- 15, 25: Inner through-hole,
- 16: Biologically active agent release layer,
- 17: Mesh opening (Communication hole),
- 41: Swelling layer,
- 42: Surface lubricating layer,
- 50, 60: Lumen, and
- 51, 61: Granulation.

## Claims

1. A stent comprising:
an outer annular body having an annular cross section, being formed with an outer through-hole penetrating in a direction that intersects with the radial direction of the annular cross section, and being capable of expanding and contracting in the radial direction; and
an inner annular body disposed inside the outer through-hole, forming a double annular cross section together with the outer annular body at least at an opening of the outer through-hole, and formed with an inner through-hole penetrating in a direction that intersects with the radial direction of the annular cross section,
wherein when placed indwelling in a living body, the outer annular body can expand in the radial direction and can thereby support a living tissue, with at least an opening of the inner through-hole of the inner annular body spaced from the living tissue.

2. The stent according to claim 1, wherein the inner annular body protrudes from the opening of the outer through-hole.

3. The stent according to claim 1 or 2, wherein the outer annular body has a communication hole through which the inside and the outside of the outer through-hole communicate with each other in the radial direction, and a swelling layer which is swellable with water is disposed at an outer surface of the inner annular body.

4. The stent according to any one of claims 1 to 3, comprising
a surface lubricating layer which is swellable with water and is disposed at an inner surface of the inner annular body.

5. The stent according to any one of claims 1 to 4, comprising
a biologically active agent release layer which contains a biologically active agent, a biodegradable polymer, and a plasticizer and which is formed on at least one of the outer annular body and the inner annular body.

6. The stent according to any one of claims 1 to 5, wherein the outer annular body and the inner annular body are each formed from a biodegradable polymer.

7. The stent according to any one of claims 1 to 6, comprising
a protrusion formed at an outer surface of the outer annular body.
